# EUROPÄISCHE PATENTSCHRIFT

(11) **EP 0 694 613 B1**
(45) Veröffentlichungstag und Bekanntmachung des Hinweises auf die Patenterteilung: **13.05.1998**
(21) Anmeldenummer: 95110058.5
(22) Anmeldetag: 28.06.1995
(51) Int. Cl.: C12N 15/86, C12N 5/10, C07K 14/00

(54) **Plasmid und damit transfizierte Zellen**
Plasmid and transfected cello therewith
Plasmide et cellules transfectées avec celui-ci

(30) Priorität: 30.07.1994 DE 4427117
(43) Veröffentlichungstag der Anmeldung: 31.01.1996
(73) Patentinhaber: GSF-Forschungszentrum für Umwelt und Gesundheit, GmbH, 85764 Oberschleissheim (DE)
(72) Erfinder: Hammerschmidt, Wolfgang, Dr., D-81247 München (DE); Sugden, Bill, Prof. Dr., Madison, WI 53707 (US)

(56) Entgegenhaltungen:
- JOURNAL OF VIROLOGY, Bd. 69, Nr. 1, 1995 Seiten 231-238, B. KEMPKES ET AL. 'Immortalization of human B lymphocytes by a plasmid containing 71 kb pairs of EBV DNA'
- PROCEEDINGS OF NATIONAL ACADEMY OF SCIENCES USA, Bd. 92, 1995 Seiten 5875-5879, B. KEMPKES ET AL. 'Immortalization of human primary B lymphocytes in vitro with DNA'
- JOURNAL OF VIROLOGY, Bd. 67, Nr. 8, 1993 Seiten 5068-5074, R. LONGNECKER ET AL. 'Deletion of DNA encoding the first five transmembrane domains of EBV latent membrane proteins 2A and 2B'
- NATURE, Bd. 340, 1989 Seiten 393-397, W. HAMMERSCHMIDT ET AL. 'Genetic analysis of immortalizing functions of EBV in human B lymphocytes'

## Beschreibung

Die Erfindung betrifft ein Plasmid nach dem Oberbegriff des Patentanspruchs 1, sowie dessen Verwendung.

Die rekombinante DNA-Technologie erlaubt die Herstellung von Epstein-Barr Virus (EBV) Vektoren, welche sich gut dazu eignen fremde Gene in B-Lymphozyten einzuschleusen. Dadurch ist es möglich die Erzeugung von fremden Proteinen in den transfizierten Zellen zu verbessern und/oder Arzneimittel zu entwickeln, welche mit den B-Lymphozyten wechselwirken. Darüber hinaus erlaubt dieser Ansatz, Zellen von individuellen Spendern zu etablieren, die zu gentherapeutischen Zwecken einsetzbar sind.

Aus der USP 5 194 601 ist ein Plasmid der gattungsgemäßen Art bekannt. Das dort beschriebene Plasmid kann eine Wirtszelle zwar infizieren, jedoch die Zelle alleine nicht immortalisieren. Hierzu sind immer Helferviren nötig, wodurch die so immortalisierten Zellen immer mit diesen Viren infiziert sind.

EBV mit einer Genomgröße von 172 kb ist in [1] beschrieben. Menschliche B-Lymphozyten, die mit EBV immortalisiert werden, enthalten mehrere extrachromosomale Kopien dieses zirkulären viralen Genoms.

Aufgabe der Erfindung ist es, ein Plasmid der e. g. Art zur Verfügung zu stellen, mit dem es gelingt, virusfreie immortalisierte Zellen zu erzeugen, sowie dessen Verwendung.

Gelöst wird diese Aufgabe durch die Patentansprüche 1, 4 bis 6.

Die Unteransprüche beschreiben vorteilhafte Ausgestaltungen des Plasmids.

Ein großer Vorteil des Plasmids besteht darin, daß bei Verwendung von damit transfizierten B-Lymphozyten oder deren Vorgängern zur Erzeugung von Proteinen, die z. B. als Medikamente verwendet werden, in diesen keine infektiösen Viren enthalten sind. Diese Viren müßten andernfalls wegen ihres Gefährdungspotentials durch aufwendige Reinigungsprozeduren entfernt werden, wodurch die Proteine zum Teil denaturiert werden können. Durch die gentechnologische Gestaltung des Plasmids ist es möglich, für die verschiedensten Anwendungen eine größere Anzahl von Basenpaaren oder Gensequenzen, bis zu einer Gesamtlänge von 25000 Basenpaaren hineinzuklonieren. Als mögliche Gene kommen z. B. verschiedene Interleukine (IL-2, IL-4; IL-7); das Gen MDR-1 (multiple drug resistance type 1), Adenosindeaminase, Immunglobuline, Tumoroberflächenantigene, Gene der Blutgerinnungsfaktoren u. a. in Betracht.

Die Identifikation einer nur beschränkten Anzahl viraler Gene, welche in EBV immortalisierten Zellen exprimiert werden, bildet die Ausgangsbasis für die Erkenntnis, welche virale Information für die Immortalisierung von B - Zellen erforderlich ist. Durch genetische Analyse dieser Gene konnte indirekt gezeigt werden, daß einige von ihnen für die Immortalisierung notwendig sind. Es ist aber unmöglich nachzuweisen, ob die so gefundenen Gene auch hinreichend für eine B-Zellen Immortalisierung sind.

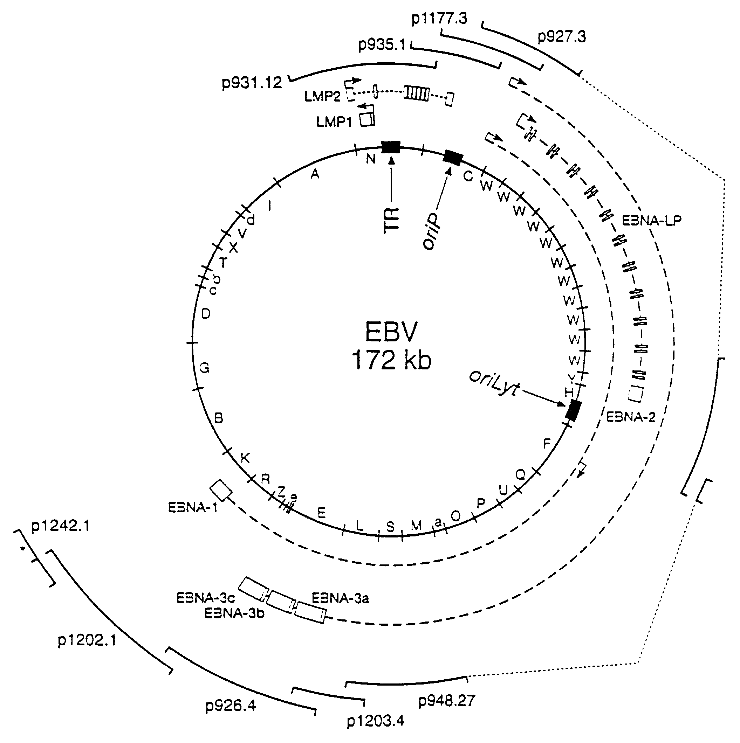

Die Erfindung wird im folgenden anhand eines Beispiels mit Hilfe der Figur näher erläutert. Dabei zeigt die Figur das Diagramm eines Plasmids.

Um herauszufinden, welcher Mindestsatz von EBV Genen für die Zellimmortalisierung ausreicht, wurde ein völlig anderer Weg beschritten. Zwei nicht zusammenhängende Teile des Genoms des EBV Stammes B95-8 [1] wurden in ein E. coli Plasmid, welches auf einem rekombinanten F-Faktor basiert, mit einer in [4] beschriebenen neuen Methode hineinkloniert. Die beiden verbundenen Teile beinhalten zusammen 71000 Basenpaare des Genoms von EBV. Das E. coli Plasmid, im folgenden p1244.8a genannt, enthält oriP und oriLyt, den latenten , bzw. den lytischen Ursprung der DNA Replikation, die Verpackungssignalsequenzen TR und alle 11 Gene, welche in immortalen B-Zellinien exprimiert sind. Einschließlich des prokaryonten Plasmidgerüsts und eines Markergens besteht die Plasmid DNA aus etwa 83000 Basenpaaren. Dies ist in etwa die halbe Größe des prototypischen EBV Stammes B95-8 [4]. Das beispielhafte prokaryonte Plasmidgerüst kann auch noch weitere Gene wie Resistenzgene enthalten. Die in cis wirkenden viralen Elemente von p1244.8a erlauben seine Vermehrung in geeigneten Helferzellen und seine Verpackung als Dimer in einer EBV Hülle. Derart verpackte Konstrukte, auch mini EBV's genannt, sind für menschliche primär B-Lymphozyten infektiös. Sie sind nützliche Vektoren für diese Zellen, die sonst nur sehr schwer DNA aufnehmen.

Die Tabelle zeigt die Plasmide, mit deren Hilfe das mini EBV Plasmid erzeugt wurde.

Die Namen der Plasmide, die EBV Teile mit ihren Nukleotidkcordinaten [1], sind zusammen mit den Restriktionsenzym-Positionen für die Subklonierung der B95.8 DNA angegeben. Der Klonierungsvektor pMBO 96 [4] wurde für alle Konstrukte benutzt außer für p931.12, welcher in pMBO132 [4] hineinkloniert wurde. Die Gene und cis-aktiven Elemente, welche für die Untersuchungen benötigt wurden, sind ebenfalls angegeben. Das Plasmid p12448.a enthält B95-8 EBV DNA von den Nukleotidpositionen #163473 bis #56081 einschließlich der verschmolzenen Enden bei Position #172272 und #1 und von Position #79656 bis #113282.

Zum Nachweis der erwünschten Wirkung des Plasmids wurde wie folgt verfahren.

Das mini EBV Plasmid p1244.8a wurde in eine Helferzellinie gebracht, welche von Burkitt'schen Lymphomen stammt, welche mit dem nicht immortalisierenden EBV Stamm P3HR1 [3] latent infiziert sind. Gleichzeitig wurde die lytische Phase des Viruszyklus durch Ko-Transfektion mit dem BZLF1 Gen [2] induziert. Die viralen Partikel, welche von den transfizierten Zellen freigesetzt wurden, enthielten DNA des P3HR1 Genoms und verpackte Dimere des mini-Plasmids. Diese Virusprodukte wurden benutzt, um primäre humane B Zellen zu infizieren. Immortalisierte B-Zell Klone wuchsen aus infizierten, jedoch nicht aus nicht infizierten Zellen. Das p1244.8a sollte sich extrachromosomal als Plasmid in infizierten Zellen replizieren, da es für EBNA1 kodiert, welches für die Plasmid Replikation erforderlich ist. Durch vergleichende Untersuchungen mit B-Zellinien, die nur mit p1244.8a infiziert waren, konnte gezeigt werden, daß das mini Plasmid p1244.8a alle viralen Informationen enthält, die notwendig sind, um B-Lymphozytenklone immortal zu erhalten. Zusätzlich konnte bewiesen werden, daß durch direkte Transfektion von mini-EBV DNA in primäre, menschliche B-Lymphocyten es möglich ist, B-Lymphozytenklone zu immortalisieren, ohne das Plasmid p1244.8a oder ähnliche über eine Helferzellinie verpacken zu müssen.

Es ist uns zum ersten Mal gelungen, mini-EBV Plasmid DNA in primäre humane B-Lymphozyten zu transferieren und damit durch direkten DNA Transfer im mortalisierte B-Zellinien zu etablieren. Die Vorteile des direkten DNA Transfers von mini-EBV Plasmid DNA wie p1244.8a und ähnlichen Plasmiden in B-Lymphozyten ergeben sich aus folgenden Punkten:
- Der direkte DNA Transfer ist experimentell einfacher als das Verpacken der Plasmide in Helferzellinien.
- Der direkte DNA Transfer erlaubt den gleichzeitigen Transfer von mehreren, auch verschieden zusammengesetzten mini-EBV Plasmiden in eine B-Zelle, was bei der Infektion von in EBV Partikel verpackte mini-EBV Plasmide nicht möglich ist.
- Durch das Verpacken der mini-EBV Plasmide befinden sich Helfervirus und rekombinante Viren, die aus Helfervirus DNA und der mini-EBV Plasmid DNA hervorgegangen sind, im Überstand, mit dem dann B-Lymphozyten infiziert werden. Diese Mischung von verschieden zusammengesetzten Viren führt zu verschiedenen B Zell Klonen, die u. U. erst durch ein genaue Analyse der entstandenen B-Zellinien zu verifizieren ist. Mit dem direkten DNA Transfer entfällt diese Schwierigkeit.
- Die Infektion von Zellen durch verpackte mini-EBV Plasmide kann nur dann erfolgreich sein, wenn der entsprechende zelluläre Rezeptor auf der zu infizierenden Zelle vorhanden ist. Das ist zwar bei B-Lymphozyten der Menschen der Fall, limitiert aber die möglichen Anwendungen auf eben diese Zellpopulation. Der direkte DNA Transfer umgeht die Notwendigkeit eines entsprechenden Rezeptormoleküls und erlaubt demnach eine Anwendung auch bei Nicht-B-Lymphozyten, z. B. T-Lymphozyten oder Fibroblasten des Menschen.

Dem mini Plasmid p1244.8a fehlen 4 von 6 wesentlichen Genen für die DNA Replikation und eine große Anzahl viraler Strukturgene, einschließlich dem wesentlichen viralen Verpackungsprotein, welche für die Virusproduktion zwingend notwendig sind. Daher können Zellen, die p1244.8a DNA alleine enthalten, keine EBV Teile erzeugen. Die Konstruktion des mini EBV Plasmids p1244.8a erlaubt in einfacher Weise weitere Modifikationen einschließlich Mutation und Aufnahme zusätzlicher Gene. Damit wird sowohl die genetische Analyse des EBV als auch die Möglichkeit bestimmte Gen in humane primäre Lymphozyten oder deren Vorgänger-von individuellen Spendern einzubringen wesentlich erleichtert.

Auf folgende Sequenzen kann vermutlich verzichtet werden, ohne daß das Plasmid seine Eigenschaft zur Immortalisierung verlieren würde.
EBV Sequenzen von Position 1 bis 7315
EBV Sequenzen von Position 56084 bis 91917
EBV Sequenzen von Position 95369 bis 97750
EBV Sequenzen von Position 110028 bis 113282,
die die Gene, die unter Patentansprüche 2. aufgeführt sind, betreffen.

### Zitierte Literatur

[1] Baer et al., (1984). DNA sequence and expression of the B95-8 Epstein-Barr virus genome. Nature, 310, 207-211.
[2] Hammerschmidt et al., (1988). Identification and characterization of *oriLyt,* a lytic origin of DNA replication of Epstein-Barr virus. Cell, 55, 427-433.
[3] Heston et al., (1982). New Epstein-Barr virus variants from cellular subclones of P3J-HR-1 Burkitt lymphoma. Nature, 295, 160-163.
[4] O'Connor et al., (1989). Construction of large DNA segments in Escherichia coli. Science, 244, 1307-1312.

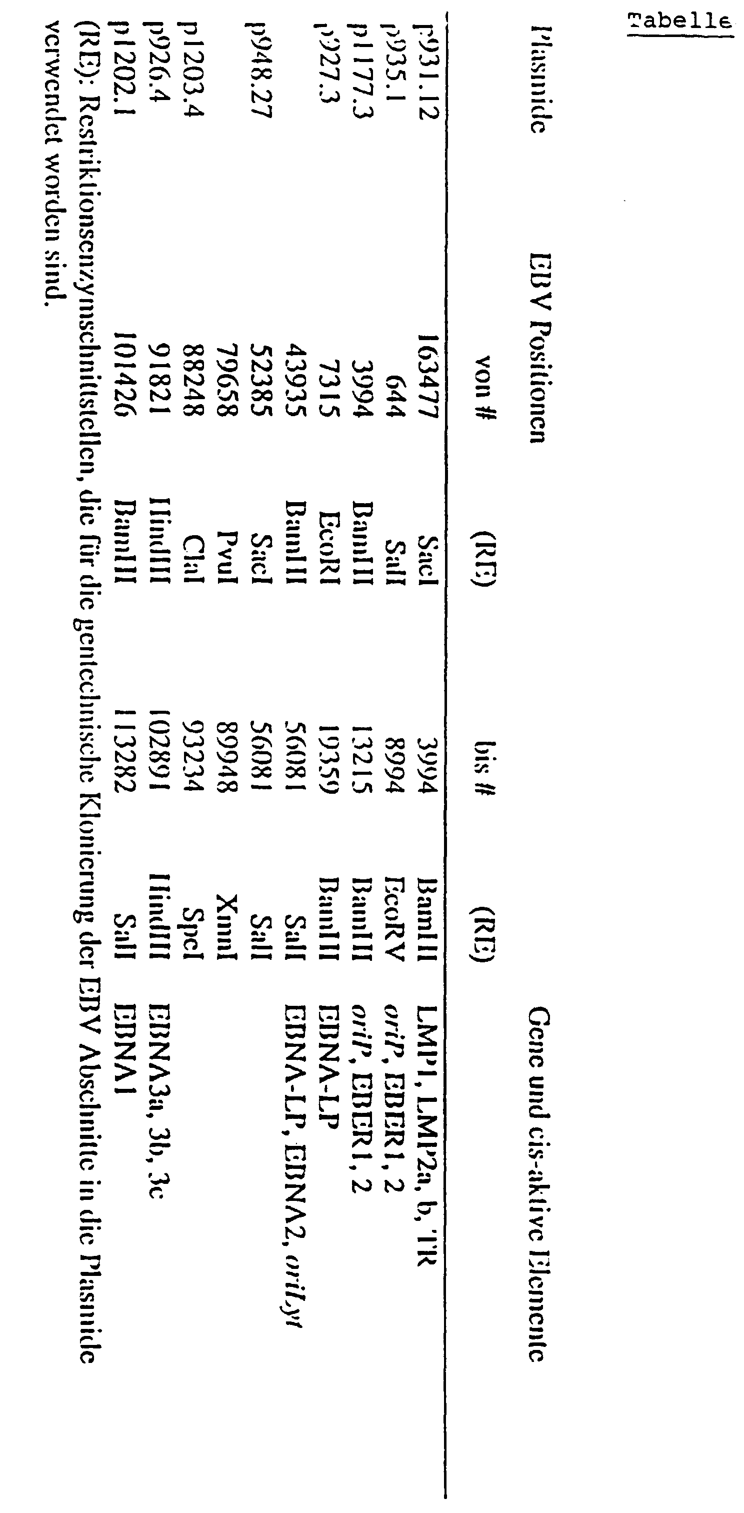

## Patentansprüche

1. Plasmid bestehend aus Teilen des Epstein-Barr Virus (EBV) und Teilen von prokaryonten Vektoren, dadurch gekennzeichnet, daß von den EBV-Genen enthalten sind:
a) LMP1 von Position 169546 bis Position 166950,
b) EBNA2 von Position 48504 bis Position 49946,
c) EBNA3a von Position 92238 bis Position 95248,
d) EBNA3c von Position 98323 bis Position 101420 und
e) EBNA1 von Position 107950 bis Position 109872.

2. Plasmid nach Anspruch 1, dadurch gekennzeichnet, daß es noch wenigstens eines der folgenden Gene des EBV enthält:
f) LMP2a von Position166498 bis Position 5856
g) LMP2b von Position 169740 bis Position 5856
h) EBER1 von Position 6629 bis Position 6795
i) EBER2 von Position 6956 bis Position 7128
j) EBNA-LP von Position 11305 (alternativ 14384) bis Position 47999
k) EBNA3b von Position 95353 bis Position 98244

3. Plasmid nach Anspruch 1 oder 2, dadurch gekennzeichnet, daß es zusätzlich bis zu 25000 Basenpaare enthalten kann, welche z. B. für Proteine kodieren.

4. B-Lymphozyren und deren Vorläufer welche das Plasmid gemäß Anspruch 1, 2 oder 3 enthalten.

5. Zellen nach Anspruch 4, dadurch gekennzeichnet, daß sie von Menschen oder von Primaten stammen.

6. Verwendung der Zellen gemäß Anspruch 4 oder 5 zur Erzeugung von Proteinen.

## Claims

1. Plasmid, comprising parts of the Epstein-Barr Virus (EBV) and parts of procaryotic vectors, characterised in that, of the EBV genes, the following are contained:
a) LMP1 from position 169546 to position 166950;
b) EBNA2 from position 48504 to position 49946;
c) EBNA3a from position 92238 to position 95248;
d) EBNA3c from position 98323 to position 101420; and
e) EBNA1 from position 107950 to position 109872.

2. Plasmid according to claim 1, characterised in that it still contains at least one of the following EBV genes:
f) LMP2a from position 166498 to position 5856;
g) LMP2b from position 169740 to position 5856;
h) EBER1 from position 6629 to position 6795;
i) EBER2 from position 6956 to position 7128;
j) EBNA-LP from position 11305 (alternatively 14384) to position 47999;
k) EBNA3b from position 95353 to position 98244.

3. Plasmid according to claim 1 or 2, characterised in that it may additionally contain up to 25000 base pairs which, for example, encode for proteins.

4. B-lymphozyrene and its precursors which contain the plasmid according to claim 1, 2 or 3.

5. Cells according to claim 4, characterised in that they originate from humans or from primates.

6. Use of the cells according to claim 4 or 5 to produce proteins.

## Revendications

1. Plasmide formé de parties du virus d'Epstein-Barr (EBV) et parties de vecteurs procaryotes
caractérisé en ce qu'
ils renferment des gènes d'EBV
a) LMP1 de la position 169546 à la position 166950,
b) EBNA2 de la position 48504 à la position 49946,
c) EBNA3a de la position 92238 à la position 95248,
d) EBNA3c de la position 98323 à la position 101420 et
e) EBNA1 de la position 107950 à la position 109872.

2. Plasmide selon la revendication 1,
caractérisé en ce qu'
il renferme encore au moins un des gènes suivants de EBV
f) LMP2a de la position 166498 à la position 5856
g) LMP2b de la position 169740 à la position 5856
h) EBER1 de la position 6629 à la position 6795
i) EBER2 de la position 6956 à la position 7128
j) EBNA-LP de la position 11305 (inversement 14384) à la position 47999
k) EBNA3b de la position 95353 à la position 98244.

3. Plasmide selon la revendication 1 ou la revendication 2,
caractérisé en ce qu'
il peut renfermer en supplément jusqu'à 25000 paires de base qui codent par exemple pour des protéines.

4. Lymphocytes B et leurs précurseurs qui renferment le plasmide selon la revendication 1, 2 ou 3.

5. Cellules selon la revendication 4,
caractérisées en ce qu'
elles proviennent d'humains ou de primates.

6. Utilisation des cellules selon la revendication 4 ou 5 en vue de l'obtention de protéines.
